# EUROPEAN PATENT SPECIFICATION

(11) **EP 0 897 760 B1**
(45) Date of publication and mention of the grant of the patent: **02.07.2003**
(21) Application number: 98830455.6
(22) Date of filing: 27.07.1998
(51) Int. Cl.: B07C 5/34, B65G 47/64, B65G 47/71, B65G 47/244

(54) **Conveying device in a container inspection system**
Trnasportvorrichtung in einem Behälterinspektionssystem
Dispositif de convoyage dans un système d'inspection de réceipients

(30) Priority: 29.07.1997 IT PR970043; 19.11.1997 IT PT970067
(43) Date of publication of application: 24.02.1999
(73) Proprietor: Lottici, Marco, 43013 Langhirano (Parma) (IT)
(72) Inventor: Lottici, Marco, 43013 Langhirano (Parma) (IT)
(74) Representative: Gotra, Stefano

(56) References cited:
- DE-U- 29 600 902
- FR-A- 2 437 616
- FR-A- 2 639 621
- US-A- 4 915 237

## Description

The present invention relates to a conveying device in a container inspection system. In systems for inspecting containers, in particular glass bottles, it is often necessary to check the bottom of the container while the containers continously advance on a conveyor.

Three different types of devices are known: a first device, illustrated in US-A-4,691,231, shows containers conveyed on a transparent belt on which they rest with their base, but the transparent belt is subject to wear and scratching which impair the transparency thereof and may result in reading errors in the station inspecting the bottom.

A second device for checking the bottom is known from EP-A-0,124,164 which, in the bottom inspection zone, shows the bottles kept in the suspended condition by pairs of continuous belt conveyors so that the main conveyor belt is interrupted in this zone and there is a vertical free passage for the inspection light ray.

A similar device is illustrated in EP-B-0415154 which also performs checking of the side surface of the containers prior to and after interruption of the main conveyor and in said interruption uses continuous belt conveyors with different speeds so as to produce a rotation of the containers during their forwards movement.

This second type of device has the drawback that it requires essentially three separate conveyors, namely the conveyor for the interruption section and the two conveyors respectively for the section upstream and the section downstream of said interruption, with obvious complications from the point of view of the movement and the synchronization of the conveyors.

Moreover, the interruption zone and the conveyors are positioned so that any stoppage of the middle conveyor causes a stoppage of the entire line since it is not possible to pass from the upstream conveyor to the downstream conveyor, bypassing the interruption zone.

A third device consists of a single conveyor of the rectilinear type which envisages, in zone where inspection of the bottom of the containers is performed, operation of a curved deviation apparatus which forces the containers to be conveyed, in the suspended condition, along a curved path outside the conveyor and then return onto said conveyor once inspection of the bottom has been completed.

The deviation apparatus, which consists of a star-wheel conveyor which grips the containers by the heck and/or by the body, or of lateral guides which create an obligatory path for the containers, are however not an effective solution and it is not desirable that the containers should pass from a rectilinear conveying movement to a conveying movement along a curved path, which is difficult to adjust in the event of a change in the shape or size of the containers.

Moreover said deviation apparatus cannot be bypassed in the case where inspection is not required or the inspection device is faulty and does not allow the containers to be brought into a storage zone downstream of the inspection zone.

The devices according to the known art have, moreover, in most cases, the additional drawback of division of the rectilinear conveyor belt, namely the rectilinear belt is composed of two sections, one upstream and one downstream of the inspection zone and this makes it necessary to provide two drive systems: one for the belt downstream and one for the rectilinear belt upstream of the deviation, and moreover in the vicinity of the end zone of the rectilinear section, bending of the conveyor links is often the cause of sudden movements of the containers.

The object of the present invention is that of eliminating the abovementioned drawbacks and providing a conveying device which allows inspection of the bottom, while keeping the containers in the suspended condition with the bottom being able to be inspected in a perfect manner.

A further object is that of achieving this in an extremely simple and low-cost manner and such that it may be disconnected from the rest of the plant in the event that inspection of the bottom is not required.

Another object is that of avoiding sudden movements of the containers along the various conveying sections.

In systems for inspecting containers, there is also the need to be able to analyse the side wall of the container from various angles in order to eliminate possible errors due to shadow zones or particular reflections.

A first solution, which is rather costly, envisages the use of several telecameras which detect the image of the container while the latter advances on a rectilinear conveyor belt.

In other solutions, which use a single telecamera, it is indispensable to have mirrors which are positioned so as to reconstruct the image of the side surface of the container or which perform rotation of the container during inspection.

In order to produce this rotation it is known to use two side belts which are movable at different speeds and between which the advancing containers are gripped so that they rotate during their forwards movement.

Another solution envisages the use of a single movable side belt and a fixed wall: the containers gripped between fixed wall and movable belt advance rotating about themselves.

The abovementioned solutions require, however, a drive system for operating the belt or the belts and therefore envisage the use of complex kinematic mechanisms.

A further solution envisages the use of a single-path rectilinear conveyor which widens, producing an automatic rotation of containers during their forwards movement and positioning in an adjacent zig-zag configuration similar to a quincuncial arrangement.

This solution has the drawback, however, that it requires a certain thrust applied to the containers themselves and is not activated for the first containers, i.e. until the outlet channel, where the containers proceed again along a single path in a straight row, is fully occupied. Moreover, it is difficult to control the amount of the angle of rotation.

In the various examples mentioned above it is possible to perform inspection by means of one or more telecameras which detect images in successive steps so as to acquire different view-points of the same container.

FR 2639621 discloses a well known conveyor of the type single/multiple-path provided with a deviation device to convey the containers in one of the three outlet lines. This device can not perform an inspection of the containers in suspended condition.

US 4915237 is another well known device of single-path type wherein the containers are inspected in a suspended condition. However, this device has the drawbacks of the prior art already cited in the application, because it needs two separated conveyors (one before and one after the inspection section) with separated motorisations and it can not avoid the suspended inspection zone.

The object of the present invention is to eliminate the possible drawbacks of the solutions mentioned above and in particular to provide a device which is able to produce a controlled and adjustable rotation of the containers in an extremely simple and low-cost manner, also applicable to numerous inspection systems which already exist.

Said objects are fully achieved by the device according to the present invention which is characterized by the contents of the claims indicated below.

This characteristic feature, along with others, will emerge more clearly from the following description of a preferred embodiment illustrated, purely by way of a non-limiting example, in the accompanying illustrative plates, in which:

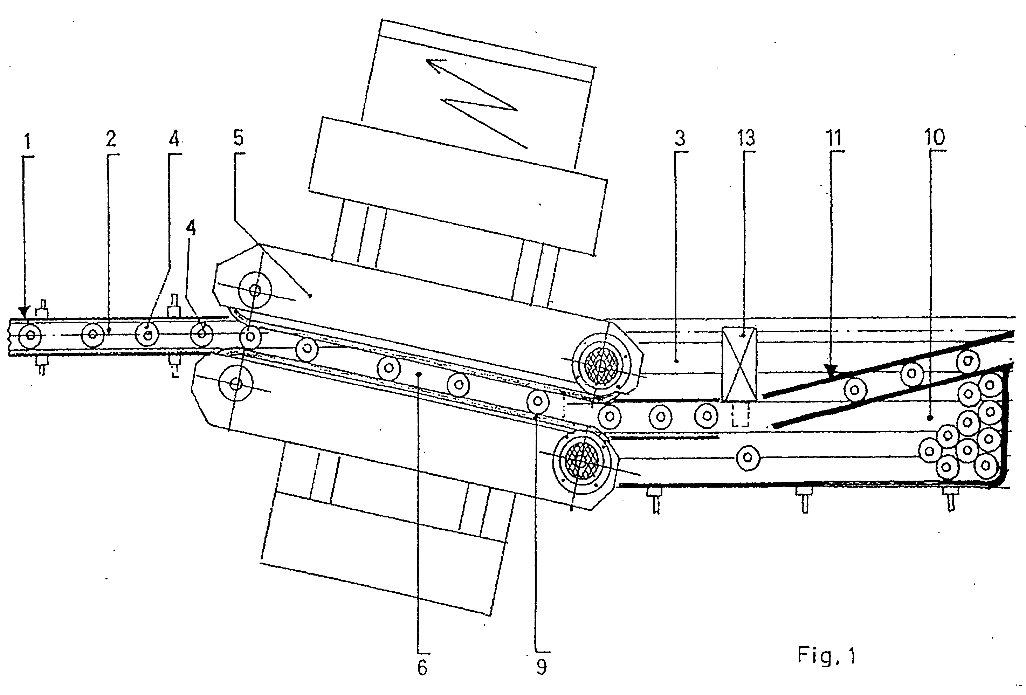

- Figures 1 and 2 show a plan view of the present device respectively in the active position for deviation and inactive position;
- Figure 3 shows a front view of the device;
- Figures 4 and 5 show a perspective view of a detail of the device, in the open position and working position, respectively;
- Figures 6 and 7 show a side view and a plan view with particular reference to the part which performs rotation of the containers;
- Figures 8 and 9 show a front view and a plan view of the same device applied to a system for inspecting full and labelled containers;
- Figure 10 shows a variation of embodiment of the device.

With reference to the Figures, 1 denotes a conveyor 1 comprising a first single-path travel section 2 and a second multiple-path travel section 3 for conveying containers 4, for example glass bottles.

The conveyor 1 is a belt conveyor of a substantially known type.

5 denotes overall a first part of a deviation conveyor which conveys the containers 4 from the first single-path travel section 2 to the second multiple-path travel section 3, causing them to pass through a zone 6 where the containers travel in the suspended condition without resting with their bottom on any conveyor.

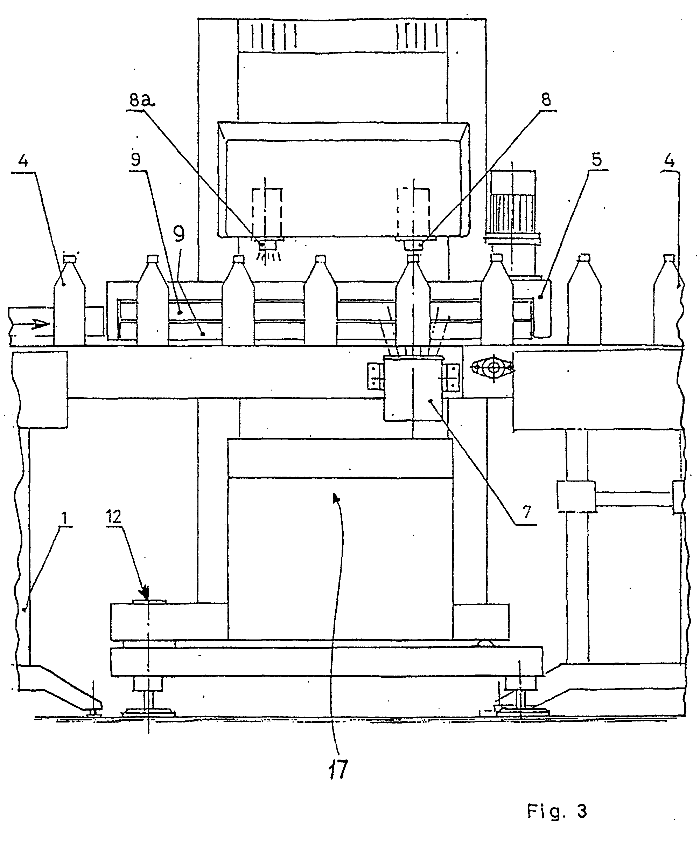

Said zone 6 is provided with a device for inspecting the bottom of the containers, consisting of an illumination device 7 and a telecamera 8, but a device for inspecting the inlet opening may also be combined therewith, said device consisting of an additional telecamera 8a provided with an illumination device in accordance with that shown in Figure 3.

The deviation conveyor in the example illustrated consists of two pairs of movable belts 9 wound on pulleys and acting on the body of the containers 4, but may also comprise a single pair of belts, and additional belts acting on the neck may be provided, or else, in accordance with a variant not illustrated, the use of belts 9 combined with a fixed wall between which the containers are retained while they advance may be envisaged.

In this latter case it is obvious that the containers, while they advance, rotate about themselves. The same result may be obtained by moving the belts 9 at different speeds or in opposite directions.

The containers 4 supplied from the first section 2 of the conveyor 1 are taken up by the deviation conveyor 5 which moves them in the suspended condition in the zone 6 where inspection of the containers occurs and then conveys them towards the second section 3 of the conveyor 1 where there is a storage area 10 created by means of a contact element 11 which may be removed if necessary.

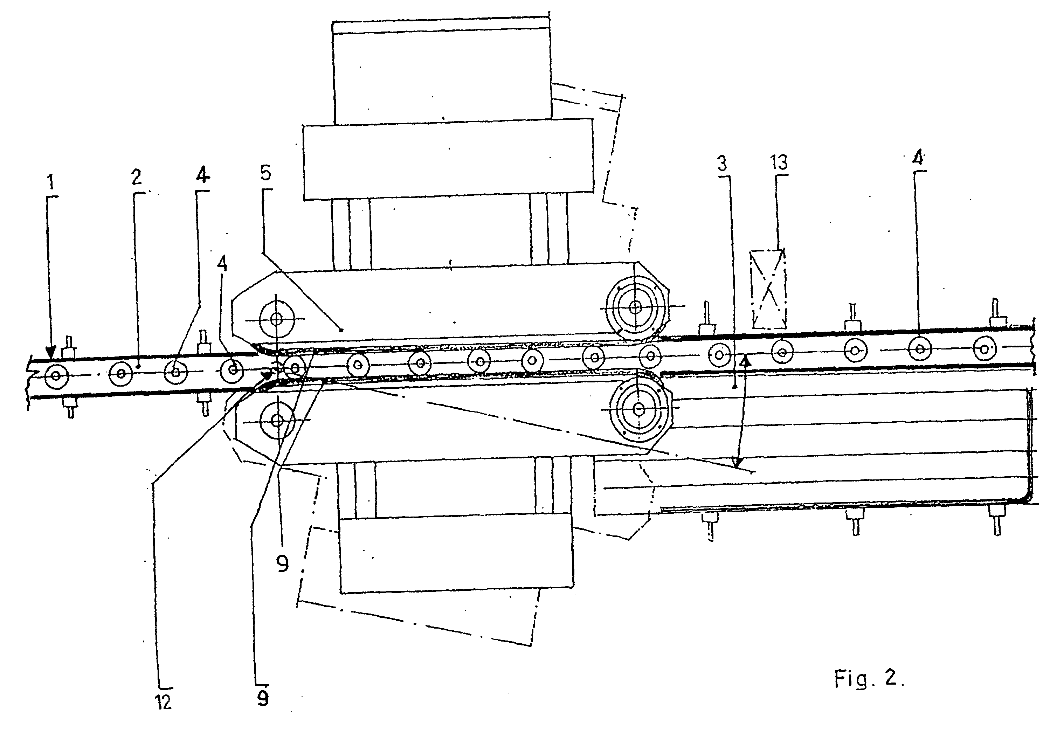

In the event of a malfunction of the deviation conveyor 5 or in the case where inspection of the containers need not be performed, the deviation conveyor 5 may be displaced, by rotating it with respect to its pivoting axis 12 so that the containers are not subject to any deviation. In this latter case, in order to increase the speed of conveying of the containers or favour their travel movement, the movable belts 9 may be widened (by means of actuator means denoted in their entirety by 14) so as to make the deviation conveyor 5 inactive: the containers are moved exclusively by the conveyor 1 and follow a rectilinear path, as illustrated in Figure 2.

In the region of the outlet of the deviation conveyor 5 into the storage area 10, a thruster device 13 may be provided, said device expelling the containers 4 considered to be defective. Obviously the thruster device 13 is controlled by a PLC connected to the device for inspecting the bottom and/or the inlet opening of the containers.

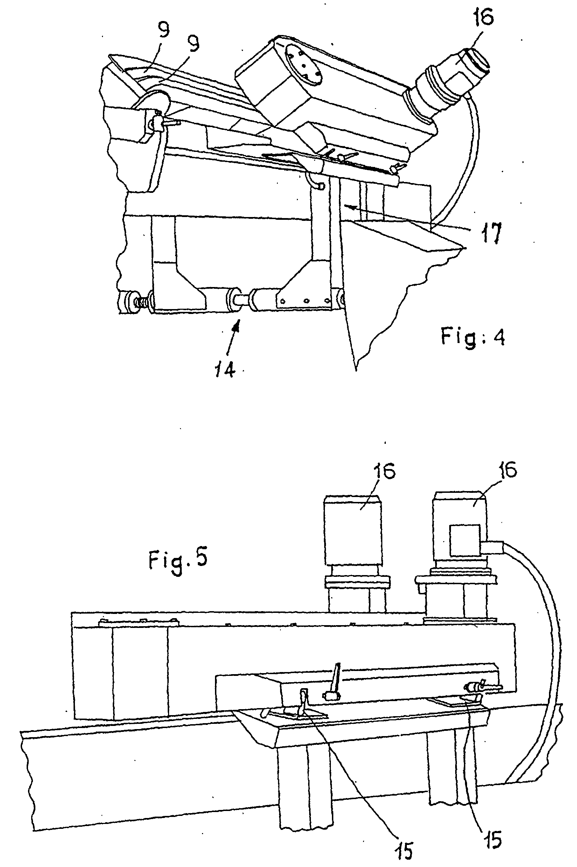

The deviation conveyor 5 is formed so as to be easily accessible for maintenance operations. In fact, as illustrated in Figure 4, the deviation conveyor 5 is composed of two parts which may open rotating about their axis via the use of a pair of hinges 15.

The two half parts are in fact hinged with a frame 17 of the device so that they may at least partially open and be folded back with a rotation about an axis parallel to the direction of conveying of the containers in the device.

16 denotes drive systems for the pulleys which move the belts 9.

The present device, which adopts an original deviation conveyor 5 which deviates laterally the containers onto a rectlinear section where they travel suspended, combines the advantages of linear travel (rapidity of change in shape or size since it is sufficient to widen the travel zone between the belts) with the advantages of the single conveyor 1 of the continuous type also in the zone where the device is applied.

This latter feature in fact allows the avoidance of sudden movements of the containers, the presence of a single drive system for the conveyor 1 and the possibility of activating lateral deviation only when it is actually required.

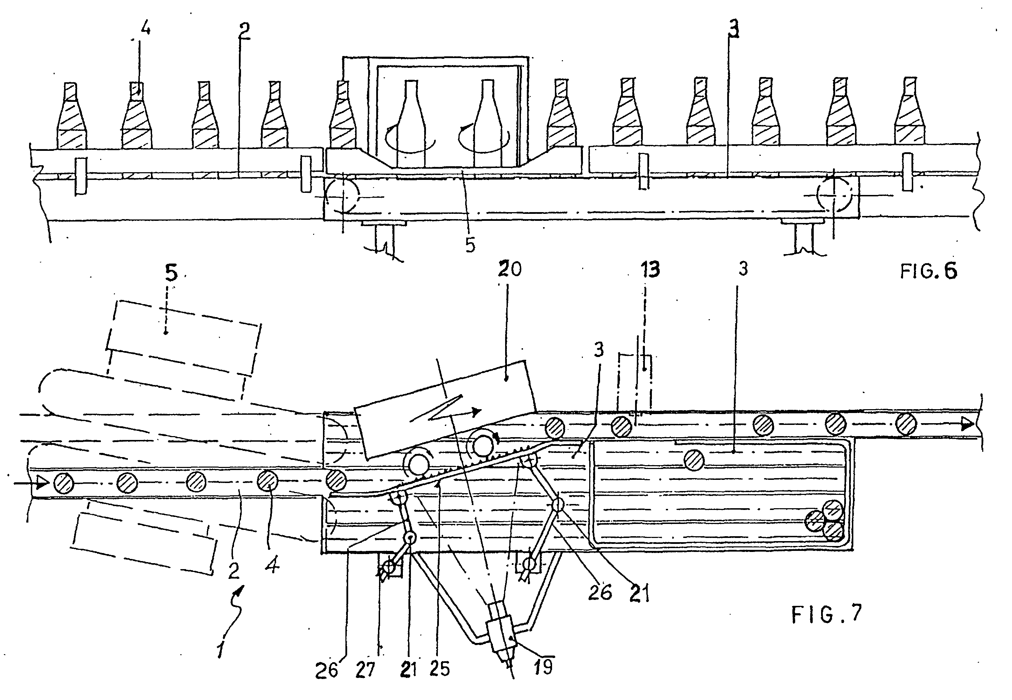

The first part of the deviation conveyor 5 may be completed, in accordance with that shown in Figures 6 to 10, by a second part 25 which conveys the containers 4 from the first travel section 2 to the second travel section 3, causing them to rotate about themselves through a certain angle.

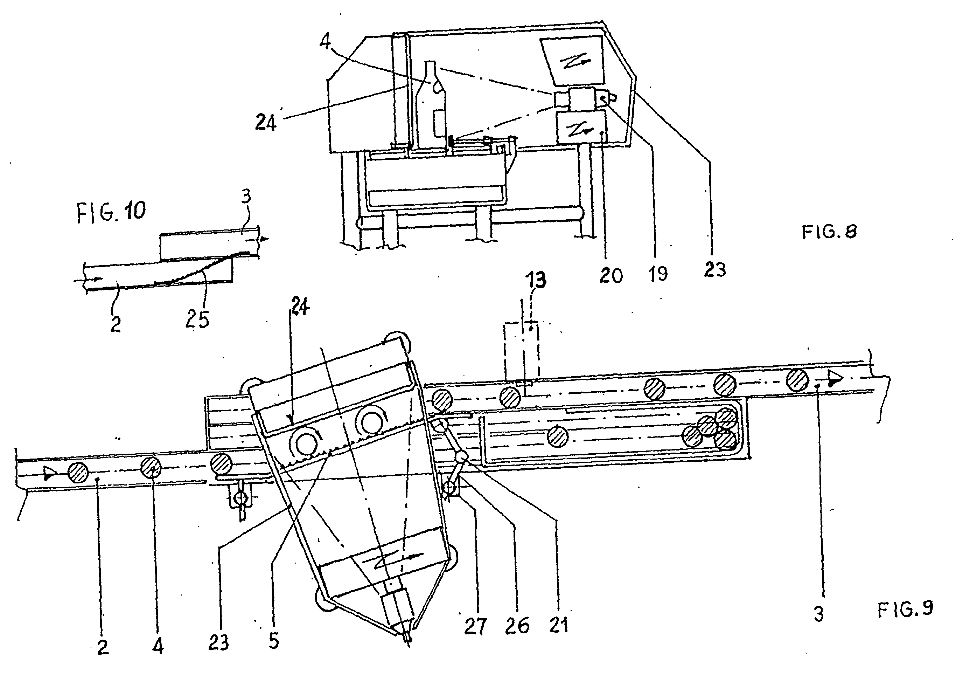

The containers 4 are supplied, in the example illustrated in Figure 7, from a bottom inspection apparatus 8 of the known type, and in particular from the first part 5 of the deviation conveyor, but may also be supplied directly by the conveyor, as illustrated for example in Figure 9.

In this case the deviation conveyor consists of a single part coinciding with the second part 25.

In the zone where the second part of the deviation device is present, inspection of the side walls of the containers and, if necessary, of the liquid contained in them is performed via means of the known type, consisting of a telecamera 19 and an illumination device 20, with the aid, where applicable (Figures 8 and 9), of a cover 23 and a background surface 24.

The present invention allows the original rotation of the containers 4 by means of simple contact and friction against the deviation conveyor during their forwards movement on the conveyor 1.

In order to facilitate said rotation, the deviation conveyor 5 comprises a shaped guide made of material which is rubbery and in any case such as to produce a sufficient friction with the containers, and the operating profile of said guide is wavy or serrated or sawtoothed.

The inclination of the guide is equal to about 10° - 35° with respect to the direction of the forwards movement of the first section 2.

The rotation of the containers is preferably through at least 90° and may be varied by varying the inclination, the length and the shape of the guide with respect to the direction of conveying into the inlet of the deviation device.

For this purpose the guide is supported by arms 26 provided with articulations 21 and secured to fixed supports 27.

By manually adjusting said arms 26 it is possible to vary the configuration and the inclination of the guide. In the example illustrated two arms 26 are present, but it is possible to envisage the use of several arms so as to allow a finer adjustment of the configuration and the inclination of the guide.

In the case illustrated in Figures 6 to 9, the second travel section 3 comprises multiple paths, each with its own speed which may be different from that of the adjacent paths.

With the variation in the speed of the individual conveying paths it is possible to vary the time required in order to perform the rotation.

Preferably the conveying paths are arranged with progressively increasing speeds in the direction of forwards movement of the containers (i.e. the conveying path closest to the outlet path has a speed which is greater than that of the conveying path adjacent to the inlet path) so as to maintain a constant thrusting force and compensate for the movement of the containers towards one another.

In a variation illustrated in Figure 10, the use of a second single-path section 3 is envisaged. In this case the inclined guide connects the two conveyors to a single path.

With the device according to the present invention it is possible to impart an automatic, controllable and adjustable rotation to the containers passing through on a conveyor during the step involving inspection of the wall or the contents, in an extremely simple and low-cost manner, which does not require any drive system or special kinematic mechanism.

The extreme simplicity of the device also makes it suitable for installation on numerous inspection systems which are already commercially available.

According to a variation of embodiment not shown, the positions of the telecamera 19 and the illumination device 20 with respect to the guide may be inverted and in particular the arms 26 may support the illumination device 20 with the guide at the base, so that the entire side surface of the containers is visible by the telecamera and is not partially covered by the guide and in such a way as to avoid additional support means for the illumination device.

## Claims

1. Conveying device in a container inspection system, of the type comprising a conveyor (1) for containers, having at least one first single-path travel section (2) and one second multiple-path travel section (3) defining at least a continuous conveyor line from the first section (2) to the second section (3) in such a way as to define a single conveyor device, a deviation conveyor (5) formed so as to convey the containers (4) from the first single-path travel section (2) to the second multiple-path travel section (3), **characterized in that** the deviation conveyor (5) is shaped in such a way as to cause the containers (4) to pass through a zone (6) where said containers (4) travel in a suspended condition without resting on their bottom.

2. Device according to Claim 1, in which said deviation conveyor (5) is formed in such a way as to be pivotably hinged at one end (12) so as to be able to assume various deviation positions and so as to be able to assume also the same direction as the conveyor (1) in the case where no deviation is required.

3. Device according to Claim 1, in which said deviation conveyor (5) comprises at least one pair of parallel conveying bands or belts (9) which act laterally on the containers (4) in the region of the body and/or the neck.

4. Device according to Claim 3, in which said bands or belts (9) travel in opposite directions or at different speeds so as to produce a rotation of the containers (4) during their forwards movement in the deviation conveyor (5).

5. Device according to any one of the preceding claims, in which an apparatus for inspecting the bottom of the containers and/or an apparatus for inspecting the inlet opening of the containers is provided in the zone where the containers travel in a suspended condition.

6. Device according to Claim 1, in which the deviation conveyor (5) comprises two half parts hinged with a frame (17) of the device itself in such a way that they can be folded back and open at least partially with a rotation about an axis parallel to the direction of conveying of the containers (4) in the deviation conveyor (5).

7. Device according to Claim 1, wherein a further deviation conveyor (25) is provided causing the containers (4) to rotate about themselves through an angle which depends on the inclination, the length and the shape of the further deviation conveyor (25) with respect to the direction of the first section.

8. Device according to Claim 7, in which said deviation conveyor (25) comprises a shaped guide made of material which is rubbery or in any case designed to produce the rotation of the containers (4) by means of simple contact and friction of the latter against the guide during their forwards movement on the conveyor (1).

9. Device according to Claim 7, in which said deviation conveyor (25) comprises hingeable support arms (6) which are secured to fixed supports (7), the shape and the inclination of the deviation conveyor (25) being variable by means of said hingeable arms.

10. Device according to Claim 7, in which the inclination of the deviation conveyor (1) is equal to about 10° - 35° with respect to the direction of forwards movement of the containers along the first travel section (2).

11. Device according to Claim 1, wherein actuator means (14) are provided for a widening of the movable belts (9) so as to make the deviation conveyor (5) inactive.

## Patentansprüche

1. Transportvorrichtung in einem Behälterinspektionssystem derart umfassend einen Förderer (1) für Behälter mit mindestens einem ersten Einbahnförderabschnitt (2) und einem zweiten Mehrbahnenförderabschnitt (3), wobei mindestens eine kontinuierliche Transportlinie von dem ersten Abschnitt (2) zu dem zweiten Abschnitt (3) gebildet wird, so dass eine einzige Transportvorrichtung entsteht, und einen derart ausgebildeten Umleitungsförderer (5), dass die Behälter (4) von dem ersten Einzelbahnförderabschnitt (2) zu dem zweiten Mehrbahnenförderabschnitt (3) transportiert werden, **dadurch gekennzeichnet, dass** der Umleitungsförderer (5) so ausgebildet ist, dass die Behälter (4) einen Bereich (6) durchlaufen, in dem die genannten Behälter (4) in aufgehängtem Zustand verfahren, ohne sich mit ihrem Boden abzustützen.

2. Vorrichtung nach Anspruch 1, bei dem der genannte Umleitungsforderer (5) so ausgebildet ist, dass er an einem Ende (12) schwenkbar angelenkt ist, so dass er verschiedene Umleitungsstellungen sowie dieselbe Richtung wie der Förderer (1) einnehmen kann, sofern keine Umleitung verlangt wird.

3. Vorrichtung nach Anspruch 1, bei der der genannte Umleitungsforderer (5) mindestens ein Paar parallele Transportbänder oder -riemen (9) umfasst, die seitlich auf den Körper- bzw. Halsbereich der Behälter (4) einwirken.

4. Vorrichtung nach Anspruch 3, bei dem die genannten Bänder oder Riemen (9) sich in entgegengesetzten Richtungen oder mit verschiedenen Geschwindigkeiten bewegen, um eine Drehung der Behälter (4) während deren Vorwärtsbewegung in dem Umleitungsförderer (5) zu bewirken.

5. Vorrichtung nach einem der vorhergehenden AnsprUche, bei der ein Inspektionsgerät fUr die Behälterböden bzw. ein Inspektionsgerät fUr die Eintrittsöffnung der Behälter in dem Bereich vorgesehen ist, in dem die Behälter in aufgehängtem Zustand verfahren.

6. Vorrichtung nach Anspruch 1, bei dem der Umleitungsförderer (5) zwei so an einem Rahmen (17) der Vorrichtung angelenkte Hälften umfasst, dass diese zurückgeklappt werden können und sich zumindest teilweise durch Drehung um eine parallel zu der Förderrichtung der Behälter (4) in dem Umleitungsförderer (5) angeordnete Achse öffnen.

7. Vorrichtung nach Anspruch 1, bei der ein weiterer Umleitungsförderer (25) vorgesehen ist, der die Behälter (4) um einen Winkel um sich selbst dreht, der von der Neigung, Länge und Form des weiteren Umleitungsförderers (25) zu der Richtung des ersten Abschnittes abhängt.

8. Vorrichtung nach Anspruch 7, bei der der genannte Umleitungsförderer (25) eine ProfilfUhrung umfasst, deren Material aus Gummi besteht bzw. die so ausgelegt ist, dass sie die Behälter (4) durch einfache Berührung und Reibung derselben mit der Führung während der Vorwärtsbewegung der Behälter auf dem Förderer (1) dreht.

9. Vorrichtung nach Anspruch 7, bei dem der genannte Umleitungsförderer (25) anlenkbare Stützarme (6) umfasst, die an feststehenden Stützen (7) befestigt sind, wobei die Form und Neigung des Umleitungsförderers (25) durch die genannten anlenkbaren Arme veränderbar ist.

10. Vorrichtung nach Anspruch 7, bei der der Umleitungsförderers (1) etwa 10-35° zu der Richtung der Vorwärtsbewegung der Behälter entlang dem ersten Förderabschnitt (2) geneigt ist.

11. Vorrichtung nach Anspruch 1, bei dem die Betätigungsmittel (14) der Öffnung der beweglichen Riemen (9) dienen, wodurch der Umleitungsförderer (5) unwirksam wird.

## Revendications

1. Dispositif de convoyage dans un système d'inspection de récipients, du type comprenant un convoyeur (1) de récipients, ayant au moins une première section de course à trajectoire unique (2) et une seconde section de course à trajectoire multiple (3) définissant au moins une ligne de convoyage continue de la première section (2) à la seconde section (3) de manière à définir un unique dispositif de convoyage, un convoyeur de déviation (5) conformé de manière à convoyer les récipients (4) de la première section de course à trajectoire unique (2) à la seconde section de course à trajectoire multiple (3), **caractérisé en ce que** le convoyeur de déviation (5) est conformé de manière à faire passer les récipients (4) au travers d'une zone (6) dans laquelle lesdits récipients (4) se déplacent en condition suspendue sans reposer sur leur base.

2. Dispositif selon la revendication 1, dans lequel le convoyeur de déviation (5) est conformé de manière à être fixé en rotation en une extrémité (12) afin de pouvoir assumer de différentes positions de déviation ainsi qu'une position le plaçant dans la même direction que celle du convoyeur (1) dans le cas ou aucune déviation ne soit nécessaire.

3. Dispositif selon la revendication 1, dans lequel ledit convoyeur de déviation (5) comprend au moins une paire de courroies ou bandes de convoyage (9) qui agissent latéralement sur les récipients (4) dans la région du corps et/ou du col.

4. Dispositif selon la revendication 3, dans lequel lesdites courroies ou bandes (9) ont des mouvements dans des directions opposées ou à des vitesses différentes de manière à produire une rotation des récipients (4) pendant leur mouvement vers l'avant dans le convoyeur de déviation (5).

5. Dispositif selon n'importe laquelle des revendications précédentes, dans lequel un appareil pour inspecter le fond des récipients et/ou un appareil pour inspecter l'ouverture d'entrée des récipients est prévu dans la zone dans laquelle les récipients se déplacent dans une condition suspendue.

6. Dispositif selon la revendication 1, dans lequel le convoyeur de déviation (5) comprend deux moitiés fixées à un châssis (17) du dispositif de manière à ce qu'elles puissent être rabattues et ouvertes au moins partiellement avec une rotation autour d'un axe parallèle à la direction de convoyage des récipients (4) dans le convoyeur de déviation (5).

7. Dispositif selon la revendication 1, dans lequel est prévu un autre convoyeur de déviation (25) faisant pivoter les récipients (4) sur eux-mêmes selon un angle dépendant de l'inclinaison, de la longueur et de la forme du convoyeur de déviation (25) par rapport à la direction de la première section.

8. Dispositif selon la revendication 7, dans lequel ledit convoyeur de déviation (25) comprend un guide façonné réalisé en un matériau caoutchouteux ou dans tous les cas conçu pour produire la rotation des récipients (4) au moyen du simple contact et de la friction de ces derniers contre le guide pendant leur mouvement vers l'avant sur le convoyeur (1).

9. Dispositif selon la revendication 7, dans lequel ledit convoyeur de déviation (25) comprend des bras de supports articulables (7) fixés sur des supports fixes (7), la forme et l'inclinaison du convoyeur de déviation (25) étant variables au moyen de ces bras articulables.

10. Dispositif selon la revendication 7, dans lequel l'inclinaison du convoyeur de déviation (1) varie de 10° à 35° par rapport à la direction du mouvement vers l'avant des récipients dans la première section de course (2).

11. Dispositif selon la revendication 1, dans lequel sont prévus des moyens d'actionnement (14) pour un élargissement des courroies mobiles (9) de manière à rendre le convoyeur de déviation (5) inactif.
